# EUROPEAN PATENT APPLICATION

(11) **EP 3 540 679 A1**
(43) Date of publication of application: **18.09.2019**
(21) Application number: 16918464.5
(22) Date of filing: 24.11.2016
(51) Int. Cl.: G06Q 50/02, A01K 29/00, A01K 1/00

(54) **DISEASE MANAGEMENT METHOD AND DEVICES FOR PERFORMING SAME**

(30) Priority: 10.11.2016 KR 20160149744
(71) Applicant: UlikeKorea Co., Inc, Seoul 05836 (KR)
(72) Inventor: KIM, Hee Jin, Namyangju-si Gyeonggi-do 12111 (KR)
(74) Representative: J A Kemp
(86) International application number: PCT/KR2016/013616
(87) International publication number: WO 2018/088611

(57) **Abstract**

A disease management method and apparatuses performing the disease management method are disclosed. The disease management method may include receiving bioinformation of a cow from a biosensor capsule provided in a stomach of the cow, determining a health state of the cow by analyzing the received bioinformation, and transmitting information associated with the determined health state to a user who manages the cow, in which the health state may include at least one of a breeding-related state associated with estrus and delivery of the cow, a disease state of the cow, a methane gas generation state of the cow, or a rumen activity state of the cow.

## Description

### Technical Field

The following description relates to a method of managing a disease, and apparatuses performing the method.

### Background Art

A pen generally refers to a structure built to raise livestock including chickens, ducks, pigs, and cattle, and facilitate the growth and management of livestock.

In such a pen, a large number of livestock live in herds. However, in case of an outbreak of an infectious and transmissible disease, such a group living may lead to extensive damage because all animals in a pen may be infected with the disease.

For instance, a huge number of cattle may die from some fatal, infectious diseases such as bovine spongiform encephalopathy (BSE), which is casually referred to as a mad-cow disease, each year causing heavy losses to stock farms.

### Disclosure

### Technical Goals

An aspect of the present disclosure provides technology for managing and controlling a bovine disease.

Another aspect of the present disclosure also provides technology for readily and conveniently providing a biosensor capsule that is orally administered directly to cattle without a need for an expensive gathering box and transmits bioinformation to determine a health state of the cattle.

### Technical Solutions

According to an aspect of the present disclosure, there is provided a bovine disease management method including receiving bioinformation of a cow from a biosensor capsule provided in a stomach of the cow, determining a health state of the cow by analyzing the bioinformation, and transmitting information associated with the health state of the cow to a user managing the cow. The health state may include at least one of a breeding-related state associated with estrus and delivery of the cow, a disease state of the cow, a methane gas generation state of the cow, or a rumen activity state of the cow.

The determining may include determining an estrus state or a delivery state of the cow based on angular speed information, acceleration information, and temperature information of the stomach that are included in the bioinformation.

The determining may include determining an activity state of the stomach based on the angular speed information, the acceleration information, and potential hydrogen (PH) information of the stomach that are included in the bioinformation.

The determining may include determining an amount of methane gas generated in the stomach based on methane gas generation information of the stomach that is included in the bioinformation.

The determining may include determining the disease state of the cow based on the angular speed information, the acceleration information, the temperature information, a PH level, and the methane gas generation information of the stomach that are included in the bioinformation.

The bovine disease management method may further include providing an alarm corresponding to the health state.

The information associated with the health state may include information associated with estrus, insemination, pregnancy, and delivery of the cow, and information associated with a recent estrus date, an expected insemination date, a recent insemination date, an expected examination date, an expected delivery date, an expected drying-off date, a recent delivery date, and an expected estrus date of the cow.

According to another aspect of the present disclosure, there is provided a disease management server including a communication module, and a controller configured to receive bioinformation of a cow from a biosensor capsule provided in a stomach of the cow through the communication module, determine a health state of the cow by analyzing the bioinformation, and transmitting information associated with the health state of the cow to a user managing the cow. The health state may include at least one of a breeding-related state associated with estrus and delivery of the cow, a disease state of the cow, a methane gas generation state of the cow, or a rumen activity state of the cow.

The controller may determine an estrus state or a delivery state of the cow based on angular speed information, acceleration information, and temperature information of the stomach that are included in the bioinformation.

The controller may determine an activity state of the stomach based on the angular speed information, the acceleration information, and PH information of the stomach that are included in the bioinformation.

The controller may determine an amount of methane gas generated in the stomach based on methane gas generation information of the stomach included in the bioinformation.

The controller may determine the disease state of the cow based on the angular speed information, the acceleration information, the temperature information, a PH level, and the methane gas generation information of the stomach that are included in the bioinformation.

The controller may provide an alarm corresponding to the health state.

The information associated with the health state may include information associated with estrus, insemination, pregnancy, and delivery of the cow, and information associated with a recent estrus date, an expected insemination date, a recent insemination date, an expected examination date, an expected delivery date, an expected drying-off date, a recent delivery date, and an expected estrus date of the cow.

According to still another aspect of the present disclosure, there is provided a biosensor capsule including a weight configured to allow the biosensor capsule to be provided at a predetermined location in a body of a cow, a plurality of sensors configured to obtain bioinformation of the cow, and a communication module configured to transmit the bioinformation. The communication module may be a long-range (LORA) communication module.

The sensors may include an acceleration sensor configured to sense an acceleration value of the stomach of the cow, a gyrosensor configured to sense an angular speed value of the stomach of the cow, a temperature sensor configured to sense a temperature value of the stomach of the cow, a methane sensor configured to sense an amount of methane gas generated in the stomach of the cow, and a PH sensor configured to sense a PH level of the stomach of the cow.

### Brief Description of Drawings

FIG. 1 is a diagram illustrating a disease management system according to an example embodiment.
FIG. 2 is a diagram illustrating a biosensor capsule illustrated in FIG. 1.
FIG. 3 is a diagram illustrating a disease management server illustrated in FIG. 1.
FIGS. 4A through 4D are diagrams illustrating examples of information associated with a health state of a cow that is provided by a disease management server according to an example embodiment.
FIG. 5 is a flowchart illustrating an operating method of a disease management server illustrated in FIG. 1.

### Best Mode for Carrying Out Invention

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the disclosure of this application. For example, the sequences of operations described herein are merely examples, and are not limited to those set forth herein, but may be changed as will be apparent after an understanding of the present disclosure, with the exception of operations necessarily occurring in a certain order. Also, descriptions of features that are known in the art may be omitted for increased clarity and conciseness.

The features described herein may be embodied in different forms, and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application.

Terms such as first, second, A, B, (a), (b), and the like may be used herein to describe components. Each of these terminologies is not used to define an essence, order, or sequence of a corresponding component but used merely to distinguish the corresponding component from other component(s). For example, a first component may be referred to as a second component, and similarly the second component may also be referred to as the first component.

It should be noted that if it is described in the specification that one component is "connected," "coupled," or "joined" to another component, a third component may be "connected," "coupled," and "joined" between the first and second components, although the first component may be directly connected, coupled or joined to the second component. In addition, it should be noted that if it is described in the specification that one component is "directly connected" or "directly joined" to another component, a third component may not be present therebetween. Likewise, expressions, for example, "between" and "immediately between" and "adjacent to" and "immediately adjacent to" may also be construed as described in the foregoing.

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, operations, elements, and/or components, but do not preclude the presence or addition of one or more other features, integers, operations, elements, components, and/or groups thereof.

Unless otherwise defined, all terms, including technical and scientific terms, used herein have the same meaning as commonly understood by one of ordinary skill in the art to which this disclosure pertains based on an understanding of the present disclosure. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

Hereinafter, some example embodiments will be described in detail with reference to the accompanying drawings. Regarding the reference numerals assigned to the elements in the drawings, it should be noted that the same elements will be designated by the same reference numerals, wherever possible, even though they are shown in different drawings.

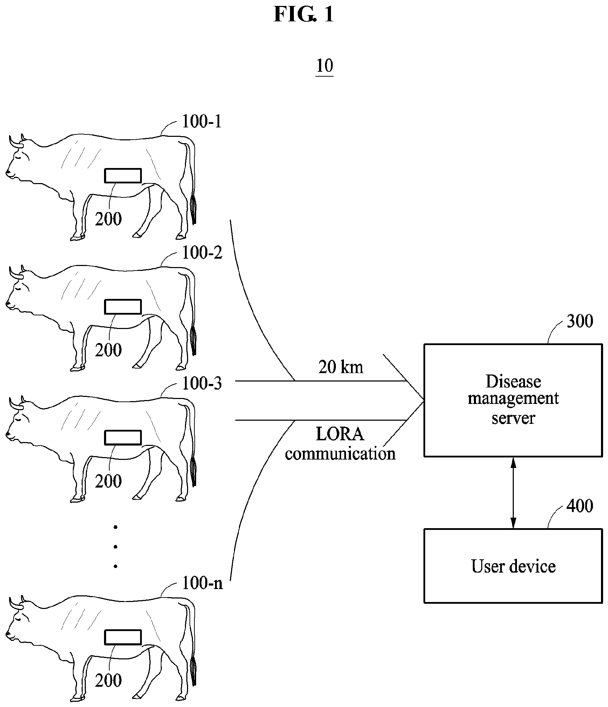

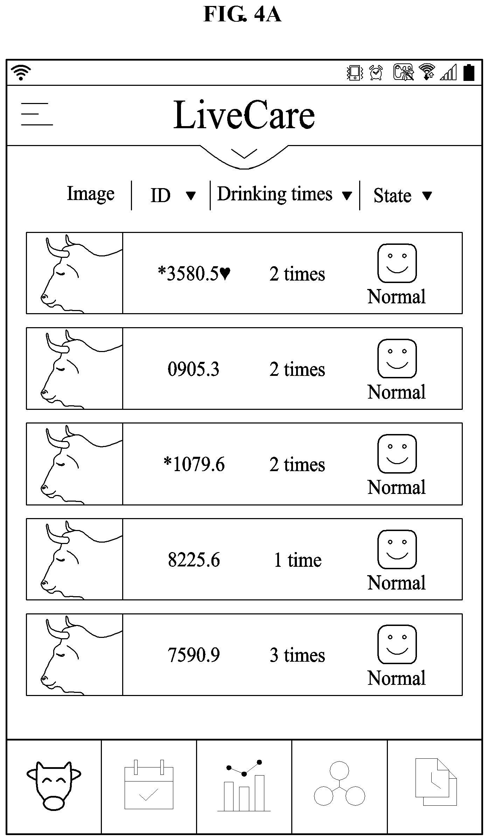

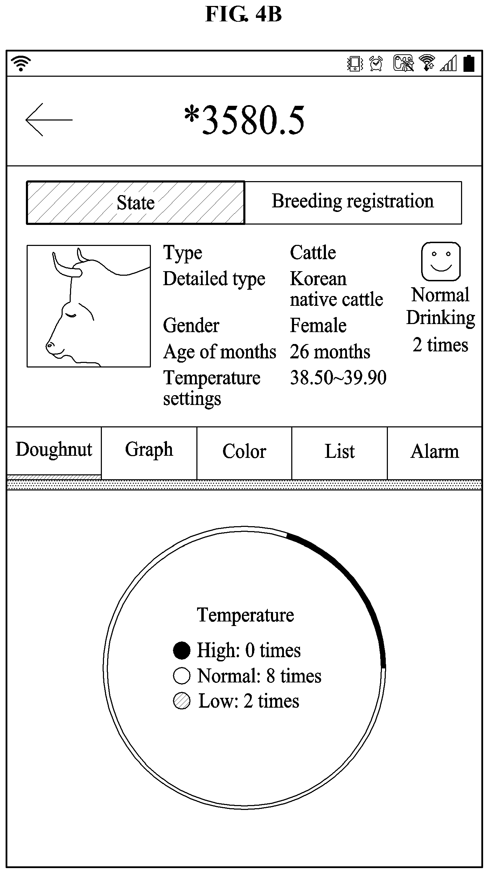

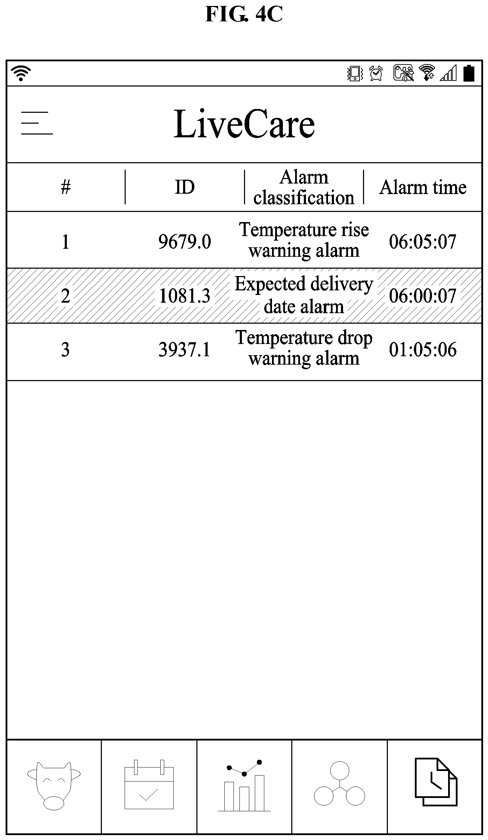

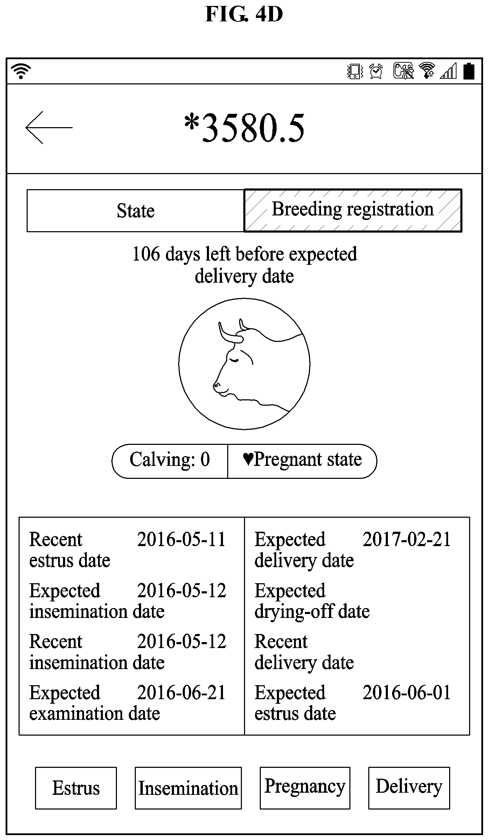

FIG. 1 is a diagram illustrating a disease management system according to an example embodiment. FIGS. 4a through 4d are diagrams illustrating examples of information associated with a health state of a cow that is provided by a disease management server according to an example embodiment.

Referring to FIG. 1, a disease management system 10 includes a plurality of cows, for example, a cow 100-1 through a cow 100-n in which n denotes a natural number greater than 1, a disease management server 300, and a user device 400.

A biosensor capsule 200 is provided in each of the cows 100-1 through 100-n. The cows 100-1 through 100-n may be managed by a user of the user device 400. In addition, some of the cows 100-1 through 100-n may be managed by another user different from the user of the user device 400.

An identification (ID) may be assigned to each of the cows 100-1 through 100-n to be managed by the disease management server 300. For example, the cows 100-1 through 100-n may be classified by each ID assigned to the biosensor capsule 200, and managed by the disease management server 300 based on the assigned ID.

The cows 100-1 through 100-n may transmit bioinformation to the disease management server 300 through the biosensor capsule 200. The biosensor capsule 200 may be placed and settled, or provided (used hereinafter), at a predetermined location in a body of each of the cows 100-1 through 100-n. Thus, the biosensor capsule 200 may obtain the bioinformation from the cows 100-1 through 100-n and transmit the obtained bioinformation to the disease management server 300.

The disease management server 300 may manage the cows 100-1 through 100-n, and provide the user device 400 with a service to remotely manage the cows 100-1 through 100-n. For example, the disease management server 300 may provide the service for management of the cows 100-1 through 100-n based on the bioinformation obtained from the biosensor capsule 200 of the cows 100-1 through 100-n.

The disease management server 300 may analyze the bioinformation, determine respective health states of the cows 100-1 through 100-n based on a result of the analyzing, and transmit the determined health states to the user device 400. Here, a health state of a cow may include at least one of a breeding-related state associated with estrus and delivery of the cow, a disease state of the cow, a methane gas generation state of the cow, or a rumen activity state of the cow.

In addition, the disease management server 300 may provide the user device 400 with an alarm that is classified based on a health state of a cow.

A disease management service application that may implement the service provided by the disease management server 300 may be installed and implemented in the user device 400, and perform a function associated with a disease management service. For example, the disease management service application may be downloaded from the disease management server 300 to be installed. For another example, the disease management service application may be downloaded from an application store or an Android market to be installed.

The user device 400 may receive, from the disease management server 300, the health states of the cows 100-1 through 100-n and/or the alarm that is classified based on a health state of a cow or corresponds to a health state of a cow. Here, information associated with a health state of a cow may include, for example, information associated with estrus, insemination, pregnancy, and delivery of the cow. The breeding-related state may include information associated with a recent estrus date, an expected insemination date, a recent insemination date, an expected examination date, an expected delivery date, an expected drying-off date, a recent delivery date, and an expected estrus date.

Examples of the information associated with the health state that is provided by the disease management server 300 to the user device 400 are illustrated in FIGS. 4a through 4d.

The user device 400 may be embodied as a personal computer (PC), a data server, or a portable electronic device.

The portable electronic device may be embodied as a laptop computer, a mobile phone, a smartphone, a tablet PC, a mobile Internet device (MID), a personal digital assistant (PDA), an enterprise digital assistant (EDA), a distal still camera, a digital video camera, a portable multimedia player (PMP), a personal or portable navigation device (PND), a handheld game console, an e-book, or a smart device. The smart device may be embodied as a smart watch, a smart band, or a smart ring.

As described, the user may remotely manage a plurality of objects, for example, the cows 100-1 through 100-n as illustrated, collectively through the user device 400 and continuously manage a history of each object, and receive information on a real-time state of each object and receive an alarm corresponding to a health state of each object to respond rapidly and accordingly.

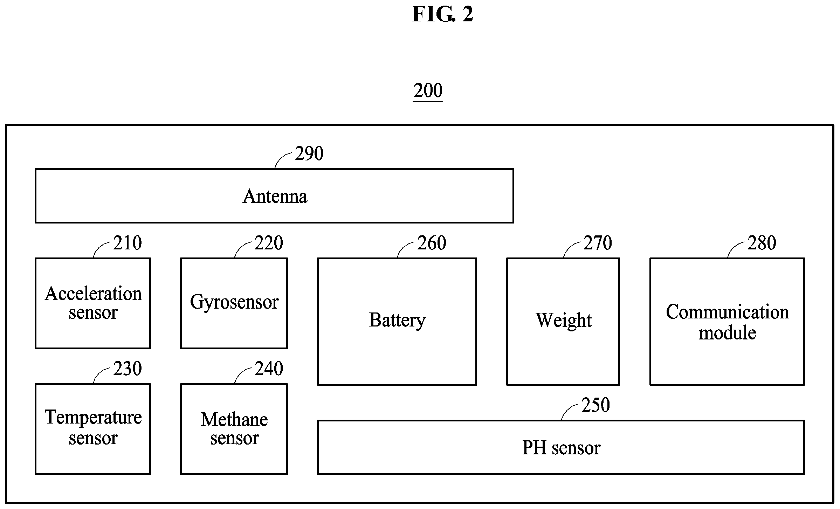

FIG. 2 is a diagram illustrating the biosensor capsule 200 illustrated in FIG. 1.

Referring to FIG. 2, the biosensor capsule 200 includes a plurality of sensors, for example, an acceleration sensor 210, a gyrosensor 220, a temperature sensor 230, a methane sensor 240, and a potential hydrogen (PH) sensor 250, a battery 260, a weight 270, a communication module 280, and an antenna 290.

The biosensor capsule 200 may be provided at a predetermined location, for example, a stomach, in a body of a cow. Here, the biosensor capsule 200 may be protected by a case (not shown) used for protection from moisture and gastric acid in the body of the cow.

The acceleration sensor 210 may obtain acceleration information of the stomach of the cow. For example, the acceleration sensor 210 may sense, in real time, an acceleration value of the stomach of the cow and generate the acceleration information.

The gyrosensor 220 may obtain angular speed information of the stomach of the cow. For example, the gyrosensor 220 may sense, in real time, an angular speed value of the stomach of the cow and generate the angular speed information.

The temperature sensor 230 may obtain temperature information of the stomach of the cow. For example, the temperature sensor 230 may sense, in real time, a temperature value of the stomach of the cow and generate the temperature information.

The methane sensor 240 may obtain methane gas generation information of the stomach of the cow. For example, the methane sensor 240 may sense, in real time, an amount of methane gas generated in the stomach of the cow and generate the methane gas generation information.

The PH sensor 250 may obtain PH information of the stomach of the cow. For example, the PH sensor 250 may sense, in real time, a PH level of the stomach of the cow and generate the PH information.

The acceleration information, the angular speed information, the temperature information, the methane gas generation information, and the PH information may be transmitted to the disease management server 300 through the communication module 280 and the antenna 290.

The battery 260 may supply power to components of the biosensor capsule 200, for example, the acceleration sensor 210, the gyrosensor 220, the temperature sensor 230, the methane sensor 240, the PH sensor 250, the communication module 280, and the antenna 290. For example, the battery 260 may be embodied in various types of battery.

The weight 270 may be provided to add a weight so that the biosensor capsule 200 is injected into the body of the cow not to be externally discharged and to be settled at a predetermined location, for example, the stomach, in the body of the cow. That is, the weight 270 may have a sufficient weight to allow the biosensor capsule 200 not to be externally discharged, but to be settled in the stomach of the cow, during a ruminating process of the cow.

Hereinafter, how the biosensor capsule 200 is not externally discharged by the weight 270 will be described as follows.

A cow is a ruminant animal that plucks grass in a rush and places the grass into a stomach without chewing the grass, and ruminates on the grass or food in a safe place, because the cow, an herbivore, is anxious about a potential attack by a carnivore.

A stomach of a ruminant animal is divided into four stomachs, as also shown in a case of a cow. The four stomachs includes a first stomach or a paunch that stores food eaten in a rush and dominates approximately 80% of a total stomach volume of a cow, a second stomach, or a reticulum or a honeycomb, that mixes the food and spits the food back to a mouth of the cow, and a third stomach, or a psalterium or a manyplies, and a fourth stomach, or an abomasum or a read, through which the food ruminated on in the mouth moves to intestines of the cow.

For example, food that is swallowed without being chewed by a cow becomes a lump to enter the first stomach or the paunch that is the largest of the four stomachs. The food becomes more wet and softer through the first stomach, and enters the second stomach or the reticulum. The food is divided into suitable size portions in the second stomach. The food is brought back from the second stomach to a mouth of the cow, and is ruminated on when the cow is free. Thus, the cow chews over and over all day. When the cow swallows again the food after chewing, the food moves to the third stomach or the psalterium and the fourth stomach or the abomasum, and finally to intestines of the cow. Through such a process, the cow digests the food.

Such a complex digestion process may take more than three days, and the cow may consume all nutrients contained in the food in the digestion process. In general, a healthy cow may start ruminating on a feed 30 to 40 minutes after the cow eats the feed, and the ruminating may be repeated six to eight times for 40 to 50 minutes. Here, the feed of approximately 50 to 60 kilograms (kg) may be ruminated on. That is, the cow may eat for eight hours, ruminate for eight hours, and rest for eight hours per day.

The biosensor capsule 200 may be injected into the body of the cow, and then be settled at a predetermined location, for example, the first stomach or the paunch, in the body of the cow. The first stomach or the paunch may dominate 80% of a total volume of the stomach of the cow, and include a plurality of spaces. Thus, the first stomach or the paunch may have a suitable space in which the biosensor capsule 200 is to be settled.

As described, a cow, which is a ruminant animal that ruminates on food, may ruminate on a considerable amount of food brought back to a mouth of the cow from the first stomach (paunch) and the second stomach (reticulum). In such a ruminating process, the biosensor capsule 200 may be mixed with the food and then discharged again to a mouth of the cow.

In addition, the first stomach or the paunch has a plurality of spaces, and food in the stomach of the cow may be mixed with water in general. Thus, when the biosensor capsule 200 is not weighed sufficiently, the biosensor capsule 200 may float above the food due to the water in such a mixing process, and be discharged to the mouth along with the food in the ruminating process.

Thus, using the weight 270, the biosensor capsule 200 may be mixed with food and move along with the food in the spaces of the stomach of the cow, and then sink to a bottom of a space by gravity and move there, and then be naturally settled at a suitable location not to be discharged outside the body and to allow a plurality of sensors, for example, the sensors 210 through 250, of the biosensor capsule 200 to accurately measure internal information of the cow.

The communication module 280 and the antenna 290 may be used to perform communication between the biosensor capsule 200 and the disease management server 300. That is, the biosensor capsule 200 and the disease management server 300 may exchange signals or data through the communication module 280 and the antenna 290.

Here, the communication module 280 may be embodied as a long-range (LORA) communication module. Thus, without a need for a gathering device between the biosensor capsule 200 and the disease management server 300, the biosensor capsule 200 may transmit the bioinformation to the disease management server 300 by accessing a communication network within a distance of 20 kilometers (km) through the communication module 280.

The sensors 210 through 250 may be integrated in a printed circuit board (PCB). In addition, the communication module 280 and/or the antenna 290 may also be integrated in the PCB.

As described, the biosensor capsule 200 may be orally administered directly to a cow, and be thus installed readily and conveniently without a need for an expensive gathering box.

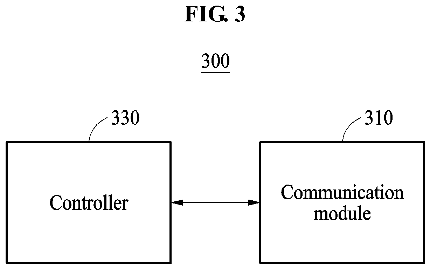

FIG. 3 is a diagram illustrating the disease management server 300 illustrated in FIG. 1.

Referring to FIG. 3, the disease management server 300 includes a communication module 310 and a controller 330.

The communication module 310 may perform communication between the disease management server 300 and the biosensor capsule 200. In addition, the communication module 310 may perform communication between the disease management server 300 and the user device 400. That is, the disease management server 300 and the biosensor capsule 200 may exchange signals or data through the communication module 310, and the disease management server 300 and the user device 400 may exchange signals or data through the communication module 310.

The controller 330 may control an overall operation of the disease management server 300. The controller 330 may receive bioinformation of a cow from the biosensor capsule 200 through the communication module 310.

The controller 330 may analyze the bioinformation, and determine a health state of the cow based on a result of the analyzing. The health state may include at least one of a breeding-related state associated with estrus and delivery of the cow, a disease state of the cow, a methane gas generation state of the cow, or a rumen activity state of the cow.

For example, the controller 330 may determine an estrus state and/or a delivery state of the cow based on angular speed information, acceleration information, and temperature information, which are included in the bioinformation.

For another example, the controller 330 may determine an activity state of a stomach of the cow based on the angular speed information, the acceleration information, and PH information, which are included in the bioinformation.

For still another example, the controller 330 may determine an amount of methane gas generated in the stomach of the cow based on methane gas generation information included in the bioinformation.

For yet another example, the controller 330 may determine the disease state of the cow based on the angular speed information, the acceleration information, the temperature information, a PH level, and the methane gas generation information, which are included in the bioinformation.

The controller 330 may transmit information associated with the health state to the user device 400 through the communication module 310. Here, information associated with the breeding-related state may include information associated with estrus, insemination, pregnancy, and delivery of the cow. In addition, the information associated with the breeding-related state may also include information associated with a recent estrus date, an expected insemination date, a recent insemination date, an expected examination date, an expected delivery date, an expected drying-off date, a recent delivery date, and an expected estrus date of the cow.

The disease management server 300 may provide the user device 400 with an alarm corresponding to the health state, for example, a temperature rise warning alarm, a temperature drop warning alarm, an alarm for the expected delivery date, an alarm for the expected examination date, an alarm for the expected drying-off date, and an alarm for the expected estrus date.

The term "module" used herein may indicate hardware that performs functions and operations corresponding to each component designated the same herein, a computer program code that performs or implements certain functions and operations, or an electronic recording medium equipped with a computer program code that performs or implements certain functions and operations, for example, a processor and a microprocessor.

The module may indicate a functional and/or structural combination of hardware to perform technical concepts or features described herein and/or software to implement the hardware.

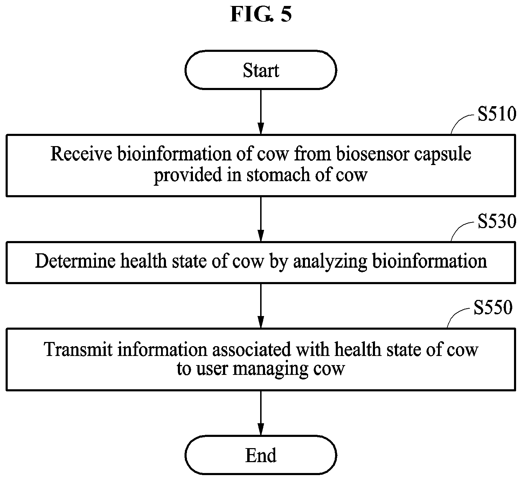

FIG. 5 is a flowchart illustrating an operating method of the disease management server 300 illustrated in FIG. 1.

Referring to FIG. 5, in S510, the controller 330 receives bioinformation of a cow from the biosensor capsule 200 provided in a stomach of the cow through the communication module 310.

In S530, the controller 330 determines a health state of the cow by analyzing the bioinformation.

In S550, the controller 330 transmits information associated with the health state of the cow to a user managing the cow through the communication module 310. The health state may include at least one of a breeding-related state associated with estrus and delivery of the cow, a disease state of the cow, a methane gas generation state of the cow, or a rumen activity state of the cow.

The units described herein may be implemented using hardware components and software components. For example, the hardware components may include microphones, amplifiers, band-pass filters, audio to digital convertors, non-transitory computer memory and processing devices. A processing device may be implemented using one or more general-purpose or special purpose computers, such as, for example, a processor, a controller and an arithmetic logic unit (ALU), a digital signal processor, a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device.

Example embodiments include non-transitory computer-readable media including program instructions to implement various operations embodied by a computer. The media may also include, alone or in combination with the program instructions, data files, data structures, tables, and the like. The media and program instructions may be those specially designed and constructed for the purposes of example embodiments, or they may be of the kind well known and available to those having skill in the computer software arts. Examples of non-transitory computer-readable media include magnetic media such as hard disks, floppy disks, and magnetic tape; optical media such as CD ROM disks; magneto-optical media such as floptical disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter. The described hardware devices may be configured to act as one or more software modules in order to perform the operations of the above-described example embodiments, or vice versa.

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents.

Therefore, the scope of the disclosure is defined not by the detailed description, but by the claims and their equivalents, and all variations within the scope of the claims and their equivalents are to be construed as being included in the disclosure.

## Claims

1. A bovine disease management method, comprising:
receiving bioinformation of a cow from a biosensor capsule provided in a stomach of the cow;
determining a health state of the cow by analyzing the bioinformation; and
transmitting, to a user managing the cow, information associated with the health state of the cow,
wherein the health state includes at least one of a breeding-related state associated with estrus and delivery of the cow, a disease state of the cow, a methane gas generation state of the cow, or a rumen activity state of the cow.

2. The bovine disease management method of claim 1, wherein the determining comprises:
determining an estrus state or a delivery state of the cow based on angular speed information, acceleration information, and temperature information of the stomach that are included in the bioinformation.

3. The bovine disease management method of claim 1, wherein the determining comprises:
determining an activity state of the stomach based on angular speed information, acceleration information, and potential hydrogen (PH) information of the stomach that are included in the bioinformation.

4. The bovine disease management method of claim 1, wherein the determining comprises:
determining an amount of methane gas generated in the stomach based on methane gas generation information of the stomach included in the bioinformation.

5. The bovine disease management method of claim 1, wherein the determining comprises:
determining the disease state of the cow based on angular speed information, acceleration information, temperature information, a PH level, and methane gas generation information of the stomach that are included in the bioinformation.

6. The bovine disease management method of claim 1, further comprising:
providing an alarm corresponding to the health state.

7. The bovine disease management method of claim 1, wherein the information associated with the health state includes:
information associated with estrus, insemination, pregnancy, and delivery of the cow; and
information associated with a recent estrus date, an expected insemination date, a recent insemination date, an expected examination date, an expected delivery date, an expected drying-off date, a recent delivery date, and an expected estrus date of the cow.

8. A disease management server, comprising:
a communication module; and
a controller configured to receive bioinformation of a cow from a biosensor capsule provided in a stomach of the cow through the communication module, determine a health state of the cow by analyzing the bioinformation, and transmitting information associated with the health state of the cow to a user managing the cow,
wherein the health state includes at least one of a breeding-related state associated with estrus and delivery of the cow, a disease state of the cow, a methane gas generation state of the cow, or a rumen activity state of the cow.

9. The disease management server of claim 8, wherein the controller is configured to determine an estrus state or a delivery state of the cow based on angular speed information, acceleration information, and temperature information of the stomach that are included in the bioinformation.

10. The disease management server of claim 8, wherein the controller is configured to determine an activity state of the stomach based on angular speed information, acceleration information, and potential hydrogen (PH) information of the stomach that are included in the bioinformation.

11. The disease management server of claim 8, wherein the controller is configured to determine an amount of methane gas generated in the stomach based on methane gas generation information of the stomach included in the bioinformation.

12. The disease management server of claim 8, wherein the controller is configured to determine the disease state of the cow based on angular speed information, acceleration information, temperature information, a PH level, and methane gas generation information of the stomach that are included in the bioinformation.

13. The disease management server of claim 8, wherein the controller is configured to provide an alarm corresponding to the health state.

14. The disease management server of claim 8, wherein the information associated with the health state includes:
information associated with estrus, insemination, pregnancy, and delivery of the cow; and
information associated with a recent estrus date, an expected insemination date, a recent insemination date, an expected examination date, an expected delivery date, an expected drying-off date, a recent delivery date, and an expected estrus date of the cow.

15. A biosensor capsule comprising:
a weight configured to allow the biosensor capsule to be provided at a predetermined location in a body of a cow;
a plurality of sensors configured to obtain bioinformation of the cow; and
a communication module configured to transmit the bioinformation,
wherein the communication module is a long-range (LORA) communication module.

16. The biosensor capsule of claim 15, wherein the sensors include:
an acceleration sensor configured to sense an acceleration value of the stomach of the cow;
a gyrosensor configured to sense an angular speed value of the stomach of the cow;
a temperature sensor configured to sense a temperature value of the stomach of the cow;
a methane sensor configured to sense an amount of methane gas generated in the stomach of the cow; and
a potential hydrogen (PH) sensor configured to sense a PH level of the stomach of the cow.
